Europäisches Patentamt

European Patent Office

Office européen des brevets

(19)

(11) Veröffentlichungsnummer: **0 177 756**
**B1**

(12) **EUROPÄISCHE PATENTSCHRIFT**

(45) Veröffentlichungstag der Patentschrift:
**02.11.88**

(51) Int. Cl.⁴: **A 61 F 2/30**, A 61 F 2/38

(21) Anmeldenummer: **85111163.3**

(22) Anmeldetag: **04.09.85**

(54) **Stiel für den Tibiateil einer Kniegelenk-Endoprothese.**

(30) Priorität: 07.09.84 DE 3432930

(43) Veröffentlichungstag der Anmeldung:
**16.04.86 Patentblatt 86/16**

(45) Bekanntmachung des Hinweises auf die Patenterteilung:
**02.11.88 Patentblatt 88/44**

(84) Benannte Vertragsstaaten:
**AT BE CH FR GB IT LI LU NL SE**

(56) Entgegenhaltungen:
**DE-B-2 660 458**

(73) Patentinhaber: **S + G IMPLANTS GMBH,
Grapengiesserstrasse 21, D-2400 Lübeck (DE)**

(72) Erfinder: **Grundei, Hans, Gärtnergasse 4, D-2400
Lübeck (DE)**

(74) Vertreter: **Wilcken, Thomas, Dipl.- Ing.,
Musterbahn 1, D-2400 Lübeck (DE)**

EP 0 177 756 B1

## Beschreibung

Die Erfindung befaßt sich mit einem Stiel für den Tibiateil einer Kniegelenk-Endoprothese. Hierzu ist es bekannt, daß die Querschnitte der beiden Schienbeine eines Menschen spiegelbildlich ausgebildet sind, wobei die Spitzen der dreieckförmigen Querschnitte winkelig zueinander und winkelig zur Gehrichtung liegen. Um daher die Stiele der Tibiateile und Kniegelenk-Endoprothesen in eine einzige drehsichere Lage einsetzen zu können, war es erforderlich, mindestens einen Längenteil des Stieles mit einem Querschnitt in Form eines unregelmäßigen Dreiecks mit abgerundeten Ecken und nach außen gewölbten Seiten zu versehen (DE-B-2 660 458). Dies führte jedoch dazu, daß für das rechte und linke Knie unterschiedliche Stiele für die Tibiateile erforderlich waren, die die Lagerhaltung äußerst stark verteuerten.

Die Aufgabe der Erfindung besteht darin, für das linke und rechte Schienbein Stiele mit gleichem Querschnitt für die Tibiateile zu gewinnen.

Diese Aufgabe wird bei einem bekannten Stiel für den Tibiateil einer Kniegelenk-Endoprothese, wobei der Querschnitt des Stieles hinten bogenförmig und vorne dachförmig ausgebildet ist, dadurch gelöst, daß das Dach als symmetrisches Spitzdach ausgebildet ist und daß der Stiel mit der Spitze des Spitzdaches in Vorwärts-Gehrichtung weisend in die Tibia implantierbar ist.

Praktisch wird damit dem Stiel ein Querschnitt erteilt, der dadurch gewonnen wird, daß die beiden bekannten Stiele für das linke und rechte Schienbein nach der DE-B-2 660 458 durch spiegelbildliches Zusammenschieben derart in Überdeckung gebracht werden, daß sie sich mit Ausnahme der beiden Dreieckspitzen überdecken, und daß sodann diese Spitzen weggeschnitten werden.

Für Implantationen kann daher dieser Tibiateil bzw. der Stiel für das linke oder rechte Knie Anwendung finden. Dadurch ist vor allen Dingen die Lagerung wesentlich vereinfacht und verbilligt. Durch das vorerwähnte gedachte Wegschneiden der Dreieckspitzen bleibt beim Implantieren auch die entsprechende Menge an spongiösem Knochen im Schienbein bestehen, so daß auch die Vorbereitung der Implantation ein geringeres Ausräumen von spongiösem Knochen erfordert.

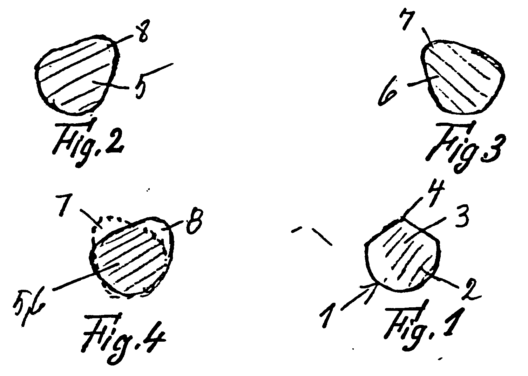

Die Erfindung wird nachstehend anhand der Zeichnung erläutert: Nach der Erfindung besitzt der Stiel 1 für den Tibiateil von Kniegelenk-Endoprothesen einen Querschnitt nach Fig. 1, der im hinteren (in der Zeichnung unteren) Teil 2 bogenförmig abweichend vom Kreis und im vorderen (in der Zeichnung oberen) Teil 3 dachförmig begrenzt mit in Gehrichtung des Patienten liegender Spitze 4 ausgebildet ist.

Mann kann sich denken, daß dieses Querschnittsprofil folgendermaßen aufgebaut ist.

Denkt man sich, daß die Querschnitte 5 und 6 (Figur 2 und 3) von Stielen für das linke und rechte Tibiateil bzw. für das linke und rechte Schienbein entsprechend der DE-B-2 660 458 Figur 2 und 5 durch spiegelbildliches Zusammenschieben in eine Überdeckung nach Figur 4 gebracht werden, so stehen dann lediglich die beiden Dreieckspitzen 7 und 8 über die Überdeckungsfläche vor. Diese spitzen Teile 7 und 8 denkt man sich weggeschnitten und erhält dann den Querschnitt des Stieles nach Fig. 1 Es ist verständlich, daß der Stiel durch dieses Profil eine Lage sowohl im linken als auch rechten Schienbein einnehmen kann.

## Patentanspruch

Stiel für den Tibiateil einer Kniegelenk-Endoprothese, wobei der Querschnitt des Stieles (1) hinten (2) bogenförmig und vorn (3) dachförmig ausgebildet ist, dadurch gekennzeichnet, daß das Dach als symmetrisches Spitzdach ausgebildet ist und daß der Stiel mit der Spitze (4) des Spitzdaches in Vorwärts-Gehrichtung weisend in die Tibia implantierbar ist.

## Revendication

Tige pour la partie de tibia d'une endoprothèse d'articulation de genou, dans laquelle la section de la tige (1) est formée en arc à l'arrière (2) et en toit à l'avant (3), caractérisée en ce que le toit est formé en toit pointu symétrique et en ce que la tige est implantable dans le tibia avec la pointe (4) du toit pointu indiquant la direction de marche avant.

## Claim

Shaft for the tibia portion of a knee-joint endoprosthesis, the cross-section of the shank being made arcuate at the back and with a corner at the front, characterized in that the corner is formed as a symmetrical angled corner and that the shaft is implantable in the tibia with the apex of the corner directed in the forward walking direction.

Fig.2

Fig.3

Fig.4

Fig.1